# EUROPEAN PATENT APPLICATION

(11) **EP 4 266 049 A1**
(43) Date of publication of application: **25.10.2023**
(21) Application number: 23165173.8
(22) Date of filing: 29.03.2023
(51) Int. Cl.: G01N 33/00, G06F 18/10, G06N 3/09, G06N 20/00

(54) **ODOR DETECTING METHOD FOR REFRIGERATING APPLIANCE AND REFRIGERATING APPLIANCE**

(30) Priority: 20.04.2022 CN 202210415781
(71) Applicant: BSH Hausgeräte GmbH, 81739 München (DE)
(72) Inventor: He, Ziyue, NANJING, 210046 (CN)

(57) **Abstract**

An odor detecting method for a refrigerating appliance and a refrigerating appliance are provided. The method includes: acquiring environmental data in the refrigerating appliance; preprocessing the environmental data, where a preprocessing method used for data in at least one dimension of the environmental data is different from a preprocessing method used for data in other dimensions; and inputting the preprocessed environmental data into a preset classification model and acquiring a classification result, where the preset classification model is configured to predict a current odor property in the refrigerating appliance according to the preprocessed environmental data. Through the solution, the obtained environmental data can be efficiently processed, and abnormal data therein is eliminated, which can help improve the classification accuracy of the odor properties in the appliance, so as to timely find and remove the peculiar smell and keep the air in the refrigerating appliance fresh more desirably.

## Description

### TECHNICAL FIELD

Embodiments of the present invention relate to the technical field of refrigerating appliances, and in particular, to an odor detecting method for a refrigerating appliance and a refrigerating appliance.

### BACKGROUND

In daily life, people use a refrigerating appliance such as a refrigerator to store perishable foods such as vegetables and fish. The refrigerating appliance extends the shelf life of food by forming a low-temperature confined space. The food placed in the refrigerating appliance inevitably produces peculiar smell. Since the compartment for storing the food is closed, the peculiar smell produced by the food cannot be dissipated autonomously, and the peculiar smell may become increasingly stronger over time. If the peculiar smell in the compartment cannot be removed in time, user experience is adversely affected. However, the prior art cannot accurately detect the peculiar smell in the refrigerating appliance and cannot remove the peculiar smell in time.

### SUMMARY

Embodiments of the present invention are intended to provide an improved refrigerating appliance and an odor detecting method for a refrigerating appliance.

Therefore, an embodiment of the present invention provides an odor detecting method for a refrigerating appliance. The method includes: acquiring environmental data in the refrigerating appliance, where the environmental data includes gas data, temperature data, and humidity data; preprocessing the environmental data, where a preprocessing method used for data in at least one dimension in the environmental data is different from a preprocessing method used for data in other dimensions; and inputting the preprocessed environmental data into a preset classification model and acquiring a classification result, where the preset classification model is configured to predict a current odor property in the refrigerating appliance according to the preprocessed environmental data.

Through the solution, the obtained environmental data can be efficiently processed, and abnormal data therein is eliminated, which can help improve the classification accuracy of the odor properties in the refrigeration appliance by the preset classification model, so as to timely find and remove the peculiar smell and keep the air in the refrigerating appliance fresh more desirably. Specifically, it is expected that environmental data including the gas data in the refrigerating appliance are collected as the input data for the model, so that the real-time odor properties in the refrigerating appliance can be accurately assessed by detecting and analyzing the gas in the refrigeration appliance. Further, according to the environmental data in different dimensions, the use of appropriate means for the preprocessing is conducive to realize the targeted processing according to the characteristics of each dimension data and improve rationality of abnormal data elimination.

Optionally, the preprocessing the environmental data includes: performing parameter scaling processing on the environmental data to unify the data in different dimensions into the value range in the same dimension, where a parameter scaling method used for the data in the at least one dimension is different from a parameter scaling method used for the data in the other dimensions; standardizing the processed environmental data so that the environmental data satisfies normal distribution; and extracting a feature of the processed environmental data to obtain the preprocessed environmental data. Since an initial order of magnitude of the environmental data in different dimensions differs greatly, which may affect a subsequent data processing, the parameter scaling method adapted to the dimensions is used in the implementation to compress the environmental data in each dimension to the same order of magnitude without changing the data features, so as to optimize the subsequent feature extraction effect.

Optionally, the performing parameter scaling processing on the environmental data to unify the data in different dimensions into the value range in the same dimension includes: taking a logarithm of values of the gas data to nonlinearly compress a range of the values of the gas data into a preset value interval; and performing maximization/minimization processing on the temperature data, the humidity data, and the gas data obtained by taking the logarithm, to obtain the processed environmental data. Therefore, the parameter scaling method adapted to the dimensions is used for compressing the environmental data in each dimension to the same order of magnitude without changing the data features.

Optionally, before the standardizing the processed environmental data, the preprocessing the environmental data further includes: eliminating abnormal data in the processed environmental data. Therefore, an impact of noise on an overall data sample is facilitated to be reduced.

Optionally, before the standardizing the processed environmental data, the preprocessing the environmental data further includes: performing interpolation processing on the processed environmental data to expand the processed environmental data. Therefore, the data sample can be effectively expanded.

Optionally, before the standardizing the processed environmental data, the preprocessing the environmental data further includes: performing data smoothing on the processed environmental data. As a result, the noise in the environmental data can be effectively processed, so that the impact of the noise on the overall data sample is reduced. Therefore, classification of a subsequent preset classification model is more accurate.

Further, the gas data includes concentration information of at least one gas component in the refrigerating appliance.

Optionally, the odor property includes at least pleasant and unpleasant. For example, a different odor property may be characterized by a score of a different value or a different interval band, and the score represents a level of the odor pleasantness within the refrigerating appliance.

Specifically, an embodiment of the present invention further provides a refrigerating appliance, which includes a gas detector, configured to collect environmental data of gas in the refrigerating appliance; a control module, configured to communicate with the gas detector to receive the environmental data, where the control module performs the odor detecting method and generates control instruction according to the current odor property in the refrigerating appliance; and a deodorization module, configured to adjust an operating parameter according to the control instruction in response to the received control instruction.

As a result, when an odor is detected based on the odor detecting method provided in the implementation, the deodorization module is actively controlled to operate and remove the odor in time.

Optionally, the refrigerating appliance further includes a communication module, where the control module communicates with the gas detector through the communication module, and/or the control module communicates with the deodorization module through the communication module.

Further features of the present disclosure are apparent from the claims, the figures and the description of figures. The features and feature combinations mentioned above in the description as well as the features and feature combinations mentioned below in the description of figures and/or shown in the figures alone are usable not only in the respectively specified combination, but also in other combinations without departing from the scope of the invention. Thus, implementations are also to be considered as encompassed and disclosed by the invention, which are not explicitly shown in the figures and explained, but arise from and can be generated by separated feature combinations from the explained implementations. Implementations and feature combinations are also to be considered as disclosed, which thus do not comprise all of the features of an originally formulated independent claim. Moreover, implementations and feature combinations are to be considered as disclosed, in particular by the implementations set out above, which extend beyond or deviate from the feature combinations set out in the back-references of the claims.

### BRIEF DESCRIPTION OF THE DRAWINGS

FIG. 1 is a flowchart of an odor detecting method for a refrigerating appliance according to an embodiment of the present invention.
FIG. 2 is a flowchart of a specific implementation of step S102 in FIG. 1.
FIG. 3 is a schematic diagram of a typical application scenario of a refrigerating appliance according to an embodiment of the present invention.

In the drawings:
1-Refrigerating appliance; 10-Compartment; 11-Gas detector; 12-Control module; 13-Deodorization module; and 14-Communication module.

### DETAILED DESCRIPTION

In order to make the foregoing objectives, characteristics, and advantages of the present invention clearer and easier to understand, specific embodiments of the present invention are described in detail below with reference to accompanying drawings.

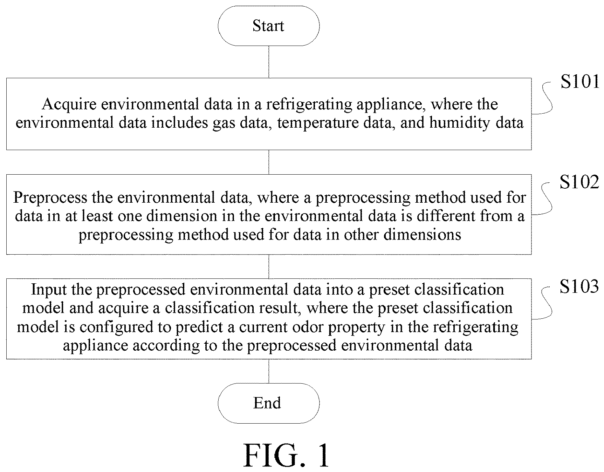

FIG. 1 is a flowchart of an odor detecting method for a refrigerating appliance according to an embodiment of the present invention.

The implementation can be applied to an odor monitoring scene in a refrigerating appliance, such as monitoring whether there is an odor in the refrigerating appliance or monitoring a real-time odor property in the refrigerating appliance. The odor property can be used for describing odor pleasantness within the refrigerating appliance, for example, including at least two categories: pleasant and unpleasant. In a practical application, a classification standard and a quantity of categories of odor properties can be adjusted as required. For example, the odor properties can be further subdivided into five categories: very pleasant, pleasant, neutral, unpleasant, and extremely unpleasant (also referred to as very unpleasant).

A different odor property may be characterized by a score of a different value or a different interval band, and the score represents a level of the odor pleasantness within the refrigerating appliance. For example, a score of 1 indicates the pleasant and a score of 0 indicates the unpleasant.

For another example, a score in a range of [0,1) indicates the unpleasant, and a score greater than or equal to 1 indicates the pleasant.

Ingredients stored in the refrigerating appliance may belong to a single category or to a plurality of categories. The implementation may specially detect the odor property of an odor produced by a specific type of ingredient in the refrigerating appliance, or may macroscopically detect the odor property of a mixed odor produced by a plurality of ingredients in the refrigerating appliance as a whole.

Specifically, referring to FIG. 1, the odor detecting method for the refrigerating appliance in this embodiment may include the following steps.

Step S101: Acquire environmental data in the refrigerating appliance, where the environmental data includes gas data, temperature data, and humidity data.

Step S102: Preprocess the environmental data, where a preprocessing method used for data in at least one dimension in the environmental data is different from a preprocessing method used for data in other dimensions.

Step S103: Input the preprocessed environmental data into a preset classification model and acquire a classification result, where the preset classification model is configured to predict a current odor property in the refrigerating appliance according to the preprocessed environmental data.

Further, the gas data includes concentration information of at least one gas component in the refrigerating appliance. A different kind of ingredient may produce a different kind of gas component under a different odor property, and an intuitive feeling of a user is to smell a different smell and then produce a different level of odor pleasure evaluation. For example, the ingredients may include meat, nuts, vegetables, and the like according to types. The odor property set may include very pleasant, pleasant, neutral, unpleasant, and extremely unpleasant. Evaluation criteria for each odor property may be determined according to sensitivity of the user to the odor, and may also be determined according to relevant industry standards.

In a specific implementation, the gas data may be acquired through a gas detector arranged in the refrigerating appliance. Specifically, step S101 may include steps: adjusting an operating temperature of the gas detector during an operation of the gas detector, and receiving candidate gas data acquired by the gas detector at each of the plurality of operating temperatures, where the candidate gas data associated with a different operating temperature is used for characterizing concentrations of different gas components; and generating the gas data based on the received plurality of candidate gas data. As a result, the operating temperature of the gas detector can be adjusted within a certain temperature range to enrich the diversity of the output data of the gas detector, so as to realize the detection of a plurality of gas components.

Further, the gas detector may include a volatile organic compounds (VOC) sensor. The VOC sensor violently reacts to specific types of gas at a different temperature, which allows the gas detector used in this implementation to detect the plurality of gas components within a certain temperature range. For example, the VOC sensor reacts with a specific type of gas component in the air and outputs a resistance value at a specific operating temperature. The resistance value can be used for characterizing concentration of the particular type of gas component.

For example, different types of ingredients may be consciously placed during an experimental phase from producing the pleasant to producing an extremely unpleasant, and the gas produced during this time may be acquired. And then, a gas analyzer is configured to analyze the gas component in the acquired gas, such as a main gas component that is present in the gas when the unpleasant is produced. The dynamic heating temperature of the VOC sensor is adjusted according to the analyzed gas component, so that the VOC sensor is more sensitive to the gas component. The adjusted VOC sensor is mounted to a suitable position on the refrigerating appliance to receive the gas data acquired by the VOC sensor in a practical application such as during performing step S101.

In a specific embodiment, the operating temperature of the gas detector may be adjusted according to a candidate value selected from a preset set of operating temperatures during the operation of the gas detector. The preset operating temperature set may include a plurality of candidate values for the operating temperature. The gas detector can accurately detect the required plurality of gas components by designing the preset operating temperature set reasonably.

Specifically, the candidate value in the preset operating temperature set may be determined according to sensitivity of the gas detector to the gas component when operating at the candidate value.

Further, a time interval for the gas detector to switch from a current operating temperature to a next operating temperature can be determined according to the time required to detect a corresponding gas component at the current operating temperature. The time required can be set in advance through the experiment.

For example, the gas data may be generated based on all candidate gas data received after traversing the preset operating temperature set at least one round, therefore, comprehensiveness of the gas data may be ensured, so that the gas data includes information on the concentration of all types of gas components that may be present in the refrigerating appliance over a period of time.

For another example, the number of candidate gas data generating the gas data may be less than the number of candidate values in the preset operating temperature set. Therefore, a response speed of gas detector is faster, and the gas data can be generated more quickly.

In a specific embodiment, each candidate value in the preset operating temperature set can be used for detecting the gas component that may be produced when a plurality of ingredients are stored together in the refrigerating appliance. Correspondingly, when performing step S101, a candidate value is selected from a single preset operating temperature set as the operating temperature of the VOC sensor, regardless of the type of ingredients currently stored in the refrigerating appliance.

In a variation, the preset operating temperature set may be specifically designed for different types of ingredients. Correspondingly, when performing step S101, the corresponding preset operating temperature set can be retrieved according to the type of ingredients currently stored in the refrigerating appliance, and the candidate value can be selected as the operating temperature of the VOC sensor.

Further, when a plurality of ingredients are stored in the refrigerating appliance at the same time, the preset operating temperature sets corresponding to the plurality of ingredients can be respectively adjusted for detection. In this example, the type of ingredients currently stored in the refrigerating appliance may be obtained from user input information or may be actively detected by a sensor device such as an image sensor.

In a specific embodiment, a temperature and humidity acquisition device can be mounted in a compartment of the refrigerating appliance to acquire temperature data and humidity data in the compartment. Alternatively, the VOC sensor can be multiplexed to acquire the temperature data and the humidity data.

In a variant, the environmental data may include gas data and operating parameters of the refrigerating appliance at the time the gas data is obtained. The operating parameters may include a compartment temperature and a compartment humidity. For example, the operating parameters can be directly obtained from a control module of the refrigerating appliance. The required environmental data predicted by the model can also be acquired, and the operating parameters are fixed values that are not affected by an external environment with opening and closing of a door switch of the refrigerating appliance. Therefore, an effect of using the operating parameters as one of the environmental data in improving the reliability of the input data is expected.

In a specific implementation, the environmental data acquired in step S101 may include raw data received from the VOC sensor. The raw data may include the resistance value outputted by the VOC sensor over a period of time, and the temperature data and the humidity data in the refrigerating appliance at the time the resistance value is generated.

Further, the preprocessing in step S 102 may include processing an original resistance value outputted by the VOC sensor, eliminating outliers in the environmental data, and so on.

An object of the preprocessing operation in step S102 may include environmental data obtained by performing step S 101 during an actual execution of the odor detecting operation of the refrigerating appliance in this implementation, and may also include a data set (including a training set, a verification set, and a testing set) used for constructing the preset classification model.

In a typical application scenario, a dynamic heating mode of the VOC sensor is assumed to have ten heating time points (referred to as heating points). When the VOC sensor is heated to these 10 heating points, the VOC sensor outputs 10 different resistance values corresponding to G0 to G9 in Table I. 0 to 9 correspond to 10 different heating points, respectively. Heating points 0 and 1 are respectively at beginning and end of an operating temperature of 50 °C, heating points 2 to 4 are between beginning and end of an operating temperature of 350 °C, heating points 5 and 6 are at beginning and end of an operating temperature of 140 °C, and heating points 7 to 9 are between the beginning and the end of the operating temperature of 350 °C. In addition, the VOC sensor can further detect the temperature and the humidity of the environment, corresponding to T (Temperature) and H (Humidity) in Table I.

**Table I Data structure outputted by VOC sensor**

| Resistance value | Temperature | Humidity |
|---|---|---|
| G0 | T0 | H0 |
| G1 | T1 | H1 |
| G2 | T2 | H2 |
| G3 | T3 | H3 |
| G4 | T4 | H4 |
| G5 | T5 | H5 |
| G6 | T6 | H6 |
| G7 | T7 | H7 |
| G8 | T8 | H8 |
| G9 | T9 | H9 |

In this scenario, the period from the beginning of heating to the end of heating is one cycle, which is about 31 seconds (s). The VOC sensor can detect the resistance value ranging from 168 ohm to 102.4 M ohm, the temperature ranging from 0 °C to 65 °C, and the humidity from 10% r.H. to 90% r.H.

In the process of data processing, since the temperature and the humidity corresponding to the 10 heating points varied very little, only T0 and H0 are extracted as calibration of the temperature and the humidity, and as output parameters to provide a raw data basis for a subsequent algorithm modeling, as shown in Table II. On the contrary, the resistance values corresponding to the 10 heating points vary widely, therefore all these 10 resistance values are used as the output parameters of the sensor to provide the raw data basis for the subsequent algorithm modeling, as shown in Table II. The parameters listed in Table II are the environmental data acquired in step S101, that is, the raw data to be preprocessed.

**Table II Parameters selection outputted by VOC sensor**

| Resistance value | | | | | | | | | | Temperature | Humidity |
|---|---|---|---|---|---|---|---|---|---|---|---|
| G0 | G1 | G2 | G3 | G4 | G5 | G6 | G7 | G8 | G9 | T0 | H0 |

In a specific implementation, after obtaining the raw data shown in Table II in step S101 and before performing step S103, step S102 can be performed to preprocess the raw data in different dimensions by using adapted means, which is conducive to making the prediction of the subsequent algorithm model more consistent with actual odor properties of the current odor in the refrigerating appliance.

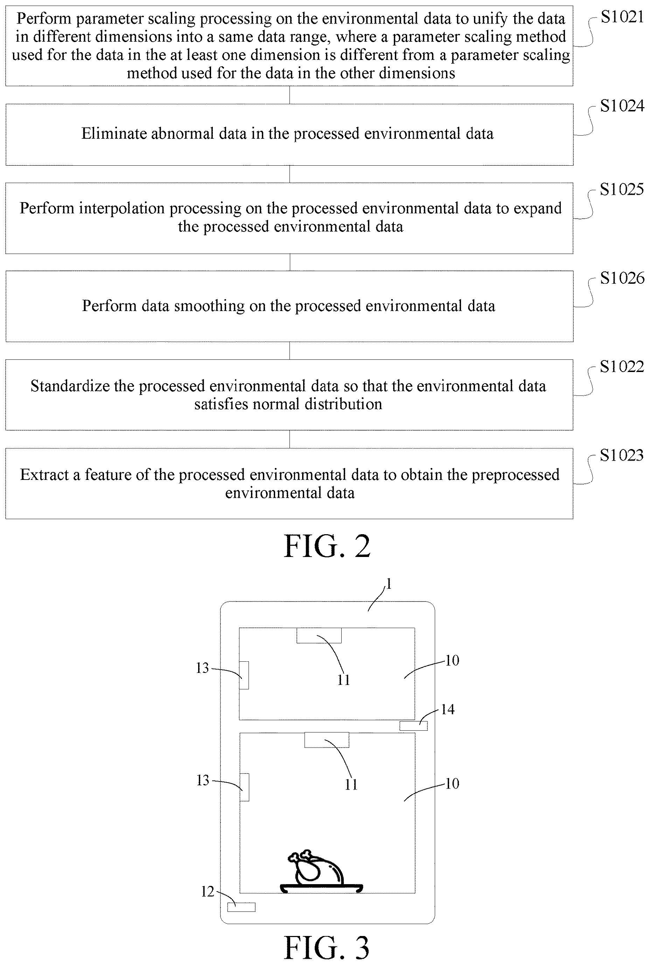

Specifically, referring to FIG. 2, step S102 may include the following steps.

Step S1021: Perform parameter scaling processing on the environmental data to unify the data in different dimensions into the value range in the same dimension, where a parameter scaling method used for the data in the at least one dimension is different from a parameter scaling method used for the data in the other dimensions.

Step S1022: Standardize the processed environmental data so that the environmental data satisfies normal distribution; and

Step S1023: Extract a feature of the processed environmental data to obtain the preprocessed environmental data.

Since an initial order of magnitude of the environmental data in different dimensions differs greatly, which may affect a subsequent data processing, the parameter scaling method adapted to the dimensions is used in step S1021 to compress the environmental data in each dimension to the same order of magnitude without changing the data features, so as to optimize the subsequent feature extraction effect.

For example, step S1021 may include steps: taking logarithmic of a value of the gas data (that is, the resistance value outputted by the VOC sensor), so as to nonlinearly compress the value range of the gas data into a preset value period; and performing maximization/minimization processing on the temperature data, the humidity data, and the gas data obtained by taking the logarithm, to obtain the processed environmental data. Therefore, the parameter scaling method adapted to the dimensions is used for compressing the environmental data in each dimension to the same order of magnitude without changing the data features.

In a practical application, the gas data may also be processed by linear compression.

The maximization/minimization processing is an implementation form of normalization processing, which may be set according to the detection capability of the gas detector, so that all environmental data given for the subsequent processing has a data range between 0 and 1.

In a specific implementation, after step S1021 and before step S1022, step S102 may further include step S1024: Eliminate abnormal data in the processed environmental data. Therefore, an impact of noise on an overall data sample is facilitated to be reduced.

For example, the abnormal data may include saturation data. The saturation data may, for example, be a resistance value that is insensitive to a change in the operating temperature. Specifically, it is found that the resistance values of G0 and G1 in Table II are usually saturated during the experiment. That is to say, the resistance values are consistently detected at 102.4 M ohm, which means that the two variables are not valid for detecting the odor changes. Therefore, the two variables can be actively discarded when the step S 1024 is performed, and the result after the elimination are shown in Table III below.

For another example, the abnormal data may include discrete data. The discrete data may, for example, be a part of the temperature data or the humidity data of the processed environmental data whose value exceeds a temperature range or a humidity range set by the refrigerating appliance.

For still another example, the abnormal data may include incomplete data. The incomplete data may, for example, be an incomplete data group after the foregoing data cleaning. In this specific implementation, the resistance value (characterizing the gas data), the temperature data, and the humidity data are recorded as a set of data group. After elimination operations of the saturation data and the discrete data, incomplete data groups are bound exist, and these incomplete data groups are required to be eliminated. In a practical application, an initial data group outputted by the gas detector is incomplete due to a stability factor of the gas detector, therefore such data is also required to be eliminated.

In a specific implementation, after step S1024 and before step S 1022, step S 102 may further include step S 1025: Perform interpolation processing on the processed environmental data to expand the processed environmental data. Therefore, the data sample can be effectively expanded.

For example, resistance values G2, G3, and G4 in Table II are resistance values measured at different heating time points at the same operating temperature and the difference between the resistance values significantly varies during the experiment. Therefore, in step S 1025, the values of G3-G2, G4-G2, and G4-G3 are added to the variable set as supplementary variables, as shown in Table III. Similarly, G6-G5, G8-G7, G9-G7, and G9-G8 are also be added to a variable set. All variables in the variable set (17 variables in total) are fed as the input data to the plurality of preset classification models for model training and testing. Different kinds of preset classification models can include classification models constructed using the same algorithm but with different parameters, or classification models constructed using different algorithms.

**Table III Preprocessed data variables**

| | |
|---|---|
| Resistance value variables | G2 |
| | G3 |
| | G4 |
| | G5 |
| | G6 |
| | G7 |
| | G8 |
| | G9 |
| Complementary resistance difference variables | G3-G2 |
| | G4-G2 |
| | G4-G3 |
| | G6-G5 |
| | G8-G7 |
| | G9-G7 |
| | G9-G8 |
| Temperature variables | T0 |
| Humidity variables | H0 |

In a specific implementation, after step S1025 and before step S 1022, step S102 may further include step S 1026: perform data smoothing on the processed environmental data. As a result, the noise in the environmental data can be effectively processed, so that the impact of the noise on the overall data sample is reduced. Therefore, classification of a subsequent preset classification model is more accurate.

The date smoothing processing can process the noise in the data, so that the impact of the noise on the overall data sample becomes less. Therefore, a subsequent training algorithm model is more accurate. In this scenario, the data smoothing method taken can take a median value of the data (including the resistance value, the temperature data, and the humidity data) every seven periods. The data of the 7 periods may be, for example, 7 groups of data variable sets shown in Table III.

After the data smoothing, part of the noise data is filtered out, fluctuation of the data is reduced, and curve becomes smoother.

In a practical application, the data smoothing processing can also be realized by averaging the data groups of the plurality of periods.

In this scenario, two groups of environmental data shown in Table I can be taken every day according to the unit of "day" and steps S 1021 to S 1026 can be performed to obtain two groups of preprocessed data variables shown in Table III. In this way, 20 groups of preprocessed data variables can be obtained after ten days, as shown in Table III. The 20 groups of preprocessed data variables can be randomly divided into the training set and the testing set in a ratio of 7:3.

The training set described in the scenario includes a training set for building a model and further includes a verification set for optimizing the model to obtain optimal parameters.

Further, when the training set and the testing set are divided proportionally, the two sets can be divided by a group of data that includes at least each ingredient or a combination of the ingredients.

Further, the data in the training set is the processed environmental data. Next, step S 1022 can be performed to standardize the processed environmental data so that all the data can satisfy normal distribution.

Further, since the final processed environmental data has a plurality of parameters (each variable can be shown in Table III), step S 1023 is further performed in this example to achieve dimension reduction through feature extraction. As a result, complexity of subsequent calculations is reduced. For example, principal component analysis (PCT) method can be used for extracting features from the processed environmental data.

In this scenario, the process of feature extraction in step S 1023 can also be understood as the process of training a normalized machine (scaler) with the training set. The testing set, as well as the preprocessed environmental data obtained by performing step S 102 during an actual implementation of the odor detection solution in the refrigerating appliance described in this embodiment, can be input into the trained normalized machine for feature extraction, and the output of the normalized machine is coupled with the preset classification model.

In a specific implementation, a model can be constructed and trained based on the training data obtained through the preprocessing of the embodiment shown in FIG. 2 and the corresponding manually calibrated odor properties, so as to obtain the preset classification model adopted in step S 103.

In a specific implementation, the environmental data acquired in step S101 can also be predicted by entering the preset classification model in step S 103 after the preprocessing operation described in the embodiment as shown in FIG. 2.

In a specific implementation, the preset classification model can at least be selected from: a K-nearest neighbor classification model (KNN classifier for short), a multi-layer neural network (MLP for short) classifier, and a support vector machine classifier (SVM classifier for short).

In a specific implementation, one the preset classification model can be arranged, and the classification result is selected from the preset odor property set. That is to say, a single preset classification model predicts, based on the input environmental data, which of the preset odor property sets the current odor property in the refrigerating appliance belongs to.

Specifically, the preset odor property set may include two kinds of odor properties: pleasant and unpleasant. Alternatively, the preset odor property set may include three kinds of odor properties: pleasant, unpleasant, and extremely unpleasant.

For example, the environmental data acquired in step S101 is fed into the preset classification model. The preset classification model outputs a score of 0 or a score of 1 or a score of 2 to characterize the results of a classification of the current odor properties within the refrigerating appliance based on environmental data. 0 can represent the pleasant, 1 can represent the unpleasant, and 2 can represent the extremely unpleasant.

Therefore, the environmental data is input into a single model for classification, and the classification result outputted by the model is the current odor property in the refrigerating appliance. An overall response speed is fast.

In a specific implementation, the preset classification model may include a first model and a second model. A classification result of the first model is selected from a first subset, and a classification result of the second model is selected from a second subset. Elements included in the first subset and the second subset are taken from the preset odor property set.

Specifically, the first subset may include one or more odor properties that represent high odor pleasantness in the preset odor property set, and the second subset can include one or more odor properties that represent low odor pleasantness in the preset odor property set.

Correspondingly, when performing step S103, combined with the current odor attributes in the refrigerating appliance determined by an odor monitoring action in the history, the environmental data acquired this time can be preferably input into the model corresponding to the subset of the current odor properties determined previously in the history, and the classification result output by the corresponding model of this subset can be obtained. If the classification result of this time tends to shift toward the odor properties included in an other subset compared with the current odor properties determined in the previous time in the history, the environmental data acquired this time is input into the model corresponding to the other subset, and the classification result output by this model is determined as a final classification result.

Therefore, by making the first model and the second model have different focuses and classifying odor properties based on corresponding subsets, the number of odor properties that require to be classified in the single model can be reduced, which may be beneficial for improving a classification accuracy of the model.

In a variant, elements of the first subset and the second subset respectively may be all the odor properties included in the preset odor property set. A difference between the two models is that parameters of the first model and the second model are different.

For example, when the preset classification model is a multi-layer neural network classification model, the difference in parameters may be for example, hidden layers of the two models adopts different activation functions, or hidden layers of the two models includes different number of neural nodes.

For another example, when the preset classification model is the K-nearest neighbor classifier, this difference in parameters may be, for example, a difference in a value of K used by the model. The value of K can be understood as follows: for a to-be-determined sample, K samples that are most similar to the samples are found from the training samples, and the be-determined sample belongs to the category in which more samples exist among these K samples.

For still another example, when the preset classification model is the SVM classifier, the difference in parameters can be caused by a difference in regularization parameters adopted by the model, or a difference in a type of kernel function adopted by the model.

Correspondingly, step S103 may include steps: inputting the environmental data into the first model and the second model, respectively, and acquiring the classification result of the first model and the classification result of the second model; and determining the classification result of the preset classification model according to an average of the classification result of the first model and the classification result of the second model, where the classification results represent the predicted odor properties with scores, and different odor properties correspond to different scores.

For example, the odor property belonging to the score closest to the average can be determined to be the current odor property within the refrigerating appliance.

Therefore, the final classification result obtained in step S103 is a product of integrating the classification results of a plurality of models, which is conducive to further improving the classification accuracy of the model.

In another variant, elements of the first subset and the second subset respectively may be all the odor properties included in the preset odor property set.

Correspondingly, step S103 may include steps: inputting the environmental data into the first model and the second model, respectively, and acquiring the classification result of the first model and the classification result of the second model. If the difference between the classification result of the first model and the classification result of the second model is greater than a first preset threshold, it indicates that this classification has low confidence. A specific value of the first preset threshold can be set as required.

When it is determined that the confidence level of this classification is low, step S101 and step S102 can be re-performed to obtain the environmental data again and input the two models for classification prediction the after preprocessing until the difference between the classification results of the two models is less than the first preset threshold, and then the final classification result is obtained based on the average of the two classification results.

Further, when it is determined that the confidence level of this classification is low, the parameters of the two models can also be iteratively updated to improve the classification accuracy.

In another variant, a sum of the elements included in each of the first subset and the second subset may be all odor properties in the preset odor property set, and the elements included in each of the first subset and the second subset may not have an intersection.

In performing step S103, the environmental data is respectively inputted into the first model and the second model. If the difference between the classification result of the first model and the classification result of the second model is less than a second preset threshold, the classification result of the first model or the classification result of the second model is determined as the final classification result of the preset classification model according to the tolerance of the user for the peculiar smell. A specific value of the second preset threshold may be set as required.

For example, it is assumed that the preset odor property set = {-1,0,1,2}, where -1 represents very pleasant, 0 represents pleasant, 1 represents unpleasant, and 2 represents very unpleasant, the first subset = {0,1} the second subset = {2,-1}, and the second preset threshold may be 1. Correspondingly, when the classification result of the first model is 0 and the classification result of the second model is -1 or when the classification result of the first model is 1 and the classification result of the second model is 2, an absolute value of the difference between the classification results of the two models is less than the second preset threshold. This means that the two models either predict the odor properties with high odor pleasantness or predict the odor properties with low odor pleasantness. In this case, if the user has high tolerance for the peculiar smell, the classification result of the first model may be determined as the final classification result. That is to say, the current odor property in the refrigerating appliance is determined as the pleasant or the unpleasant. If the user has low tolerance for the peculiar smell, the classification result of the second model may be determined as the final classification result. That is to say, the current odor property in the refrigerating appliance is determined as the very pleasant or the very unpleasant, and then the control module of the refrigerating appliance may operate the deodorization module at a higher power to increase the deodorizing strength.

If the difference between the classification result of the first model and the classification result of the second model is greater than the second preset threshold, it indicates that the two models reach opposite conclusions and this classification has low confidence. At this point, step S101 and step S102 can be re-performed to obtain the environmental data again and input the two models for classification prediction the after preprocessing until the difference between the classification results of the two models is less than the second preset threshold.

Therefore, the two models are collaboratively configured to make the classification prediction based on the environmental data, so that the accuracy of the final classification results can be expected.

In a specific implementation, the preset odor property set may include a plurality of first-level properties and a plurality of second-level properties, and the plurality of second-level properties may belong to one of the plurality of first-level properties.

Specifically, each of the first-level properties may be, for example, the unpleasant, the pleasant, or the neutral. Similar to a multi-level catalog, the second-level property may be subcategories of the first-level property. The odor monitoring scenario in the refrigerating appliance focuses on the odor property of the unpleasant, and correspondingly, a plurality of second-level properties can be set for the first-level properties of the unpleasant. These second-level properties may be, for example, ordinary unpleasant and very unpleasant.

Further, a first subset may include the plurality of first-level properties and a second subset may include the plurality of second-level properties.

Correspondingly, step S103 may include steps: inputting the environmental data into the first model and acquiring the classification result of the first model; inputting the environmental data into the second model if the classification result of the first model is a first-level property to which the multiple a second-level property belong, and acquiring the classification result of the second model; and determining the classification result of the second model as the classification result of the preset classification model.

For example, it is supposed that the preset odor property set = {0,1,2,3}, where 0 represents the pleasant, 1 represents the unpleasant, 2 represents the normal unpleasant, and 3 represents very unpleasant, the first subset = {0,1} and the second subset = {2,3}.

When the step S103 is performed, the environmental data is first input into the first model. If the classification result outputted by the first model is 0, the current odor property in the refrigerating appliance can be directly determined as the pleasant, and the odor detection in the refrigerating appliance is completed.

If the classification result outputted by the first model output is 1, it indicates that there is an odor in the refrigerating appliance. In order to further determine severity of the odor (equivalent to odor pleasantness) in the refrigerating appliance, the environmental data is input into the second model and the classification result outputted by the second model is finally determined as the current odor property in the refrigerating appliance.

It is helpful to determine whether the refrigerating appliance has an ordinary odor or very unpleasant to remove the odor from the refrigerating appliance.

Further, the first model can be trained based on the training data corresponding to the first subset, and the second model can be trained based on the training data corresponding to the second subset. It should be noted that although the training data is different, the parameters of the first model and the second model may be the same, and certainly the parameters of the two models may be different.

Therefore, the first model and the second model respectively classify the current odor properties in the refrigerating appliance at different levels based on the environmental data. Specifically, the first model can output a crude classification result based on environmental data, and the second model can output a more detailed classification result based on environmental data, so that the classification result with an appropriate subdivision can be outputted as required.

Further, the second model is configured to further refine the classification of specific odor properties, so that the first model can directly output the classification results to improve the response speed when analyzing and determining current non-specific odor properties. When the current odor properties are determined as specific odor properties, the second model is called to refine the classification, so as to obtain more accurate classification results. For example, the specific odor properties may include the unpleasant. That is to say, the level of odor pleasantness within the refrigerating appliance is below the preset threshold.

Based on the above, the implementation can efficiently process the acquired environmental data and eliminate abnormal data therebetween, which is conducive to improving classification accuracy of the preset classification model for the odor property in the refrigeration appliance, so as to timely discover and remove the odor, and better keep the air in the refrigerating appliance fresh. Specifically, it is expected that environmental data including the gas data in the refrigerating appliance are collected as the input data for the model, so that the real-time odor properties in the refrigerating appliance can be accurately assessed by detecting and analyzing the gas in the refrigeration appliance. Further, according to the environmental data in different dimensions, the use of appropriate means for the preprocessing is conducive to realize the targeted processing according to the characteristics of each dimension data and improve rationality of abnormal data elimination.

FIG. 3 is a schematic diagram of a typical application scenario of a refrigerating appliance according to an embodiment of the present invention.

Specifically, referring to FIG. 3, the refrigerating appliance 1 in this embodiment may include: a gas detector 11, configured to acquire environmental data of gas in the refrigerating appliance 1; a control module 12, communicating with the gas detector 11 to receive the environmental data, where the control module 12 performs the odor detecting method shown in FIG. 1 and FIG. 2 and generates control instruction according to the current odor property in the refrigerating appliance 1; and a deodorization module 13, where the deodorization module responds to the received control instruction and adjusts an operating parameter according to the control instruction.

For example, the refrigerating appliance 1 may include a refrigerator, a freezer, a cabinet freezer, and so on.

As a result, when an odor is detected based on the odor detecting method provided in the implementation, the deodorization module 13 is actively controlled to operate and remove the odor in time.

The gas detector 11 can be mounted in each compartment 10 of the refrigerating appliance 1 to acquire the targeted environmental data of the compartment 10 in which the gas detector is located. Alternatively, one gas detector 11 may be arranged close to a return air outlet of the refrigerating appliance 1 to collect the overall environmental data inside the refrigerating appliance 1.

The control module 12 may include a main control board of the refrigerating appliance 1. Alternatively, the control module 12 may be a module dedicated to performing the methods shown in FIG. 1 and FIG. 2.

The deodorization module 13 may be arranged in each compartment 10. For any compartment 10, when the prediction result of the environmental data obtained by the gas detector 11 of the compartment 10 by the control module 12 indicates that the odor property of the current compartment 10 is unpleasant, the deodorization module 13 of the compartment 10 operates to remove the odor of the compartment 10.

Alternatively, one deodorization module 13 may be arranged in any compartment 10, or arranged in an air duct of the refrigerating appliance 1. When the odor property of any compartment 10 is determined to be unpleasant, the deodorization module 13 operates and realizes the overall odor removal of all compartments 10 through a circulating refrigeration system in the refrigerating appliance 1.

For example, the deodorization module 13 may include an ionizer.

Limited or wireless communication can be adopted between the gas detector 11 and the control module 12, and between the control module 12 and the deodorization module 13.

Further, the refrigerating appliance 1 may include a communication module 14. The control module 12 communicates with the gas detector 11 through the communication module 14.

Further, the control module 12 and the deodorization module 13 can also communicate with each other through the communication module 14.

Alternatively, the communication module 14 can communicate with the device terminal of the user to timely inform the user of the prediction result. For example, the device terminal may include a mobile phone, an IPAD, a home monitoring device, and so on.

Further, a preset classification model may be stored in the control module 12. Alternatively, the preset classification model may be stored in other position within the refrigerating appliance 1 or outside the refrigerating appliance 1 (such as in a cloud), and the control module 12 performing the implementation can access the corresponding storage module to transmit the obtained environmental data to the module and receive the classification result of the model.

Further, the control module 12 can perform step S102 as shown in FIG. 1 to preprocess the environmental data outputted by the gas detector 11. Alternatively, the pretreatment operation described in step S102 may be performed by other specialized modules within the refrigerating appliance 1. The module communicates with the control module 12 to transmit the preprocessed environmental data to the control module 12. Alternatively, the specialized module may include the cloud. The control module 12 uploads the received environmental data to the cloud and receives the preprocessed environmental data transmitted by the cloud.

It should be noted that FIG. 5 only exemplarily shows possible arrangement positions of the control module 12, the deodorization module 13, the gas detector 11, and the communication module 14 on the refrigerating appliance 1. In practical application, mutual position relationships of the modules and the specific arrangement positions on the refrigerating appliance 1 may be adjusted as required. The modules may be independent of each other, or may be integrated in a same chip or integrated into a same functional module. For example, the control module 12 and the communication module 14 may be integrated together.

In a first application scenario, it is assumed that the preset classification model outputs "0" (pleasant) or "1" (unpleasant). Correspondingly, when "1" is outputted, the control module 12 may start the deodorization module 13 to perform a deodorization operation. With continuous or periodic monitoring of the odor in the refrigerating appliance, when the preset classification model outputs "0", the control module 12 may turn off the deodorization module 13.

In a second application scenario, it is assumed that the preset classification model outputs "0" (the pleasant), "1" (the unpleasant), or "2" (very unpleasant). Correspondingly, when "1" is outputted, the control module 12 may control the deodorization module 13 to operate at a relatively low power to perform the deodorization operation. When "2" is outputted, the control module 12 may control the deodorization module 13 to operate at a relatively high power to perform the deodorization operation. With continuous or periodic monitoring of the odor in the refrigerating appliance, when the preset classification model outputs "0", the control module 12 may turn off the deodorization module 13.

In a third application scenario, the control module 12 may store a first model and a second model. The first model outputs "0" (the pleasant) and "1" (the unpleasant), and the second model outputs "0" (an ordinary unpleasant) and "1" (very unpleasant). Correspondingly, when the first model outputs "1", the control module 12 may further input the environmental data into the second model. If the second model outputs "0", the deodorization module 13 is controlled to operate at a relatively low power to perform the deodorization operation. If the second model outputs "1", the deodorization module 13 is controlled to operate at a relatively high power to perform the deodorization operation. When the first model outputs "0", the control module 12 may turn off the deodorization module 13.

Further, in this application scenario, during the training of the first model, in addition to the training data manually labeled as the pleasant and the unpleasant, the training data manually calibrated for the very unpleasant can also be used for training the first model, so that the first model can distinguish the pleasant and the unpleasant more accurately. During the training of the second model, the training is performed based on the training data manually labeled as the unpleasant and the very unpleasant, so that the second model can accurately distinguish these two types of odor properties.

Although specific implementations have been described above, these implementations are not intended to limit the scope disclosed in the present disclosure, even if only a single implementation is described with respect to specific features. The feature examples provided in the present disclosure are intended for illustration but not limitation, unless otherwise stated. During specific implementation, the technical features of one or more dependent claims may be combined with the technical features of the independent claims, and the technical features from the corresponding independent claims may be combined in any appropriate way, not only through the specific combination listed in the claims.

Although the present invention is disclosed as above, the present invention is not limited thereto. A person skilled in the art can make various changes and modifications without departing from the spirit and the scope of the present invention. Therefore, the protection scope of the present invention should be subject to the scope defined by the claims.

## Claims

1. An odor detecting method for a refrigerating appliance, **characterized by** comprising:
acquiring environmental data in the refrigerating appliance, wherein the environmental data comprises gas data, temperature data, and humidity data;
preprocessing the environmental data, wherein a preprocessing method used for data in at least one dimension of the environmental data is different from a preprocessing method used for data in other dimensions; and
inputting the preprocessed environmental data into a preset classification model and acquiring a classification result, wherein the preset classification model is configured to predict a current odor property in the refrigerating appliance according to the preprocessed environmental data.

2. The odor detecting method according to claim 1, **characterized in that** the preprocessing the environmental data comprises:
performing parameter scaling processing on the environmental data to unify the data in different dimensions into the value range in the same dimension, wherein a parameter scaling method used for the data in the at least one dimension is different from a parameter scaling method used for the data in the other dimensions;
standardizing the processed environmental data so that the environmental data satisifes normal distribution; and
extracting a feature of the processed environmental data to obtain the preprocessed environmental data.

3. The odor detecting method according to claim 2, **characterized in that** the performing parameter scaling processing on the environmental data to unify the data in different dimensions into the value range in the same dimension comprises:
taking a logarithm of values of the gas data to nonlinearly compress a range of the values of the gas data into a preset value interval; and
performing maximization/minimization processing on the temperature data, the humidity data, and the gas data obtained by taking the logarithm, to obtain the processed environmental data.

4. The odor detecting method according to claim 2 or 3, **characterized in that** before the standardizing the processed environmental data, the preprocessing the environmental data further comprises:
eliminating abnormal data in the processed environmental data.

5. The odor detecting method according to any of the claims 2 to 4, **characterized in that** before the standardizing the processed environmental data, the preprocessing the environmental data further comprises:
performing interpolation processing on the processed environmental data to expand the processed environmental data.

6. The odor detecting method according to any of the claims 2 to 5, **characterized in that** before the standardizing the processed environmental data, the preprocessing the environmental data further comprises:
performing data smoothing on the processed environmental data.

7. The odor detecting method according to any of the preceding claims, **characterized in that** the gas data comprises concentration information of at least one gas component in the refrigerating appliance.

8. The odor detecting method according to any of the preceding claims, **characterized in that** the odor properties comprise at least pleasant and unpleasant.

9. A refrigerating appliance (1), **characterized by** comprising:
a gas detector (11), configured to collect environmental data in the refrigerating appliance (1);
a control module (12), configured to communicate with the gas detector (11) to receive the environmental data, wherein the control module (12) performs the odor detecting method according to any of claims 1 to 8 and generates a control instruction according to a current odor property in the refrigerating appliance (1); and
a deodorization module (13), configured to adjust an operating parameter according to the control instruction in response to the received control instruction.

10. The refrigerating appliance (1) according to claim 9, **characterized by** further comprising a communication module (14), wherein the control module (12) communicates with the gas detector (11) through the communication module (14), and/or the control module (12) communicates with the deodorization module (13) through the communication module (14).
